# EUROPEAN PATENT SPECIFICATION

(11) **EP 0 787 733 B1**
(45) Date of publication and mention of the grant of the patent: **05.12.2001**
(21) Application number: 97300277.7
(22) Date of filing: 17.01.1997
(51) Int. Cl.: C07D 491/048, C12P 17/18, A61K 31/44

(54) **Novel bioactive substance K93-0711 I-1 and I-2, and process for production thereof**
Bioaktive Substanz k93-0711, I-1 und I-2 und Herstellungsverfahren
Composé bioactif k93-0711, I-1 et I-2 et procédé de préparation

(30) Priority: 19.01.1996 JP 752196
(43) Date of publication of application: 06.08.1997
(73) Proprietor: THE KITASATO INSTITUTE, Tokyo 108-8641 (JP)
(72) Inventor: Omura, Satoshi, Minato-ku, Tokyo (JP); Komiyama, Kanki, Minato-ku, Tokyo (JP); Hayashi, Masahiko, Minato-ku, Tokyo (JP); Takamatsu, Satoshi, Minato-ku, Tokyo (JP)
(74) Representative: Woods, Geoffrey Corlett

(56) References cited:
- AKIRA KAWASHIMA ET AL: "Preparation of fluorinated antibiotics followed by 19F spectroscopy III. Accumulation of 3a-hydroxy-6-fluoroindoline upon addition of 6-fluorotryptophan to the cultured broth of Streptomyces sp. H-63" JOURNAL OF ANTIBIOTICS., vol. 39, no. 10, October 1986, TOKYO JP, pages 1495-1497, XP002054146
- MASAHIKO HAYASHI ET AL: "Madindoline,a novel inhibitor of IL-6 activity from Streptomyces sp. K93-0711. I.Taxonomy,fermentation,isolation and biological activities." JOURNAL OF ANTIBIOTICS., vol. 49, no. 11, November 1996, TOKYO JP, pages 1091-1095, XP002054147

## Description

The present invention relates to bioactive substance K93-0711 I-1 and 1-2 and a process for production of the bioactive substance K93-0711I-1 and I-2 which are produced by culturing microorganism having an ability to produce the same and have suppressive action for growth of IL-6 dependent MH-60 ( hereinafter designated as MH-60) cells.

Treatments for cancer are mainly classified into surgical operations, radiation therapy and chemotherapy. Progress of these therapies in the present day and new development of anticancer agents has resulted in a curative rate of cancer or upto 50%. Gastric cancer and uterine cancer are often found by progress of mass examination or periodic medical check-up and mortalities thereof are decreased. However recent changes of food and living conditions and aging society resulted to increase lung cancer, liver cancer, cancer of colon and prostatic cancer. These cancers have tendency to accompany malignancy and cause to cachexia. No effective chemotherapic agents for cancer cachexia have been found.

In progress of curative rate above 50% of cancer, importance in quality of life ( QOL ) is noticed. For example, cancerous cachexia is constant functional disorders (metabolic, endocrine and immunologic abnormalities) often observed in cancer patients, and various symptoms such as body weight loss, adipose and muscular tissue weight loss and hypercalcemia, have been observed. Cachexia causes a decrease in QOL of the patients as well as shortage of life span, increase in recurrence rate and decrease in response to anticancer agents, and results in poor prognosis. Chemotherapeutic agents with improvement action for cachexia have been expected in order to make cure and to prevent postoperative recurrence and poor prognosis.

Causes of cachexia have been unknown. However recent studies indicate that excess production of interleukin-6 (hereinafter designated as IL-6), a type of cytokine, is involved in cachexia. Therefore, a substance having inhibitory action on IL-6 activity or suppressive action as regards production of IL-6, is thought to have improved activities on cancer cachexia.

We have found that selective growth inhibition against MH-60 cells was detected in fermentation extracts of a microbial strain, K93-0711 isolated from soil. The effective substance was isolated from the cultured medium and purified. The said substance has not been reported in the references and was designed as K93-0711 I-1 and I-2. The substance has been given the generic name madindoline, refer to J. Antibiotics, 49 (11):1091-1095, 1996.

Accordingly, the present invention provides a bioactive substance which is denoted as K93-0711 I-1 or K93-0711 I-2, which is obtainable by culturing *Streptomyces sp.* K92-0711 (FERM BP-5764) and which has the following physicochemical properties:
(1) Bioactive substance K93-0711 I-1;
(i) Molecular formula : C₂₂H₂₇O₄N (high resolution FAB mass spectrum)
(ii) Molecular weight : 369 (FAB mass spectrum)
(iii) Ultraviolet and visible light absorption spectrum : UV spectrum in methanol has specific absorption maxima at 299 (log ε 3.28), 245 (log ε 4.26) and 207 (log ε 4.38) nm
(iv) Infrared absorption spectrum:
IR spectrum in KBr has absorption bands at 3390, 2927, 1741, 1695, 1603, 1487, 1381, 1281 and 756 cm⁻¹

(v) Specific rotation [α]²⁴_{D} +44.4° (c. 0.3, MeOH)
(vi) Solubility:
Soluble in acetone, ethyl acetate, ethyl ether, hexane, methanol, ethanol, chloroform and benzene
Slightly soluble in water

(vii) ¹H-NMR spectrum (in deuterated chloroform, 400 MHz) and ¹³C-NMR spectrum (in deuterated chloroform, 100.58 MHz)

| ¹³C - shift | ¹H shift(J) |
|---|---|
| 206.4 (s) | |
| 206.3 (s) | |
| 157.7 (s) | |
| 156.6 (s) | |
| 150.5 (s) | |
| 130.5 (d) | 7.19 (1H, m) |
| 129.5 (s) | |
| 123.5 (d) | 7.19 (1H, m) |
| 119.1 (d) | 6.75 (1H, td, 7.3, 0.5) |
| 108.0 (d) | 6.63 (1H, d, 8.0) |
| 106.2 (d) | 4.93 (1H, s) |
| 88.0 (s) | |

| ¹³C - shift | ¹H shift(J) |
|---|---|
| 66.6 (t) | 3.84 (1H, m) 3.14 (1H, m) |
| 53.7 (t) | 3.69 (1H, d, 14.2) |
| | 3.45 (1H, d, 14.2) |
| 50.6 (s) | |
| 41.1 (t) | 2.33 (1H, dd, 12.0, 9.0) |
| | 2.17 (1H, ddd, 12.0, 5.0, 1.5) |
| 29.9 (t) | 1.29 (2H, m) 1.29 |
| 23.5 (t) | 2.38 (1H, m) 2.36 (1H, m) |
| 22.7 (t) | 1.19 (2H, m) |
| 17.3 (q) | 1.12 (3H, s) |
| 13.8 (q) | 0.76 (3H, t, 7.0) |
| 9.4 (q) | 2.00 (3H, s) |

(viii) Color reaction : positive for sulfuric acid, Ehrlich and iodine.
(2) Bioactive substance K93-0711 I-2;
(i) Molecular formula : C₂₂H₂₇O₄N (high resolution FAB mass spectrum)
(ii) Molecular weight : 369 (FAB mass spectrum)
(iii) Ultraviolet and visible light absorption spectrum: UV spectrum in methanol has specific absorption maxima at 304 (log ε 3.28), 246 (log ε 4.29) and 208 (log ε 4.40) nm
(iv) Infrared absorption spectrum
IR spectrum in KBr has absorption bands at 3410, 2927, 1738, 1693, 1597, 1489, 1380, 1282 and 756 cm ⁻¹

(v) Specific rotation [α]²⁴_{D} +25.6° (c. 0.3, MeOH)
(vi) Solubility :
Soluble in acetone, ethyl acetate, ethyl ether, hexane, methanol, ethanol, chloroform and benzene
Slightly soluble in water

(vii) ¹H-NMR spectrum (in deuterated chloroform, 400 MHz) and ¹³C-NMR spectrum (in deuterated chloroform,100.58 MHz)

| ¹³C - shift | ¹H - shift |
|---|---|
| 206.6 (s) | |
| 205.9 (s) | |
| 160.3 (s) | |
| 153.7 (s) | |
| 150.4 (s) | |
| 130.5 (d) | 7.19 (1H, m) |
| 129.4 (s) | |
| 123.5 (d) | 7.19 (1H, m) |
| 118.9 (d) | 6.74 (1H, td, 7.2, 1.0) |
| 107.8 (d) | 6.62 (1H,d, 8.0) |
| 105.0 (d) | 4.92 (1H, s) |
| 87.9 (s) | |
| 66.8 (t) | 3.85 (1H, ddd, 12.0, 7.9, 1.5) |
| | 3.17 (1H, ddd, 12.0, 9.2, 5.0) |
| 52.1 (t) | 3.69 (1H, d, 14.9) |
| | 3.49 (1H, d, 14.9) |
| 50.5 (s) | |
| 41.2 (t) | 2.33 (1H, ddd, 12.0, 7.9, 1.5) 2.19 (1H, ddd, 12.0, 9.2 1.5) |
| 29.5 (t) | 1.39 (2H, m) |
| 24.0 (t) | 2.46 (1H, m) 2.37 (1H, m) |

| ¹³C - shift | ¹H - shift |
|---|---|
| 17.5 (q) | 1.12 (3H, s) |
| 13.8 (q) | 0.86 (3H, t, 7.1) |
| 9.1 (q) | 1.94 (3H, s) |

(viii) Color reaction : positive for sulfuric acid, Ehrlich and iodine.

The invention further provides:
- a process for production of bioactive substance K93-0711 I-1 or I-2 according to the invention, comprising culturing *Streptomyces sp.* K93-0711 (FERM BP-5764), or a mutant thereof which produces said bioactive substance K93-0711 I-1 or I-2, in a medium to accumulate said bioactive substance K93-0711 I-1 or I-2 in the medium; and isolating said bioactive substance therefrom.
- *Streptomyces sp.* K93-0711 (FERM BP-5764), or a mutant thereof which produces bioactive substance K93-0711 I-1 or I-2 according to the invention.
- a bioactive substance according to the invention for use in a method of treatment of the human or animal body by therapy.
- a pharmaceutical composition comprising a bioactive substance according to the invention as defined in claim 1 and a pharmaceutically acceptable carrier or diluent.

A preferable strain for production of bioactive substance K93-0711 I-1 and I-2 of the present invention is Streptomyces sp. K93-0711 which was isolated by the present inventors. Taxonomical properties of the strain are illustrated as follows.

### I. Morphological properties:

Vegetative mycelia grew abundantly on various agar media and showed bacillary elements. The aerial mycelia grew abundantly on oatmeal agar and glycerol-asparagine agar. The aerial mass color was white to gray. The spore chains were Retiflexibiles type and each had more than 20 spores per chain. The spores were cylindrical in shape, 1.4 X 0.7 µm in size, and had a smooth surface. Sclerotic granules, sporangia and flagellated spores were not observed.

### II. Culture characteristics on various media:

Culture characteristics of the present strain were investigated by a method of E. B. Shirling and D. Gottlieb (Int. J. Syst. Bacteriol. 16: 313-340, 1966) and results are shown in Table 1.

The Color Harmony Manual, 4th Ed., 1958 (Container Corp. of America, Chicago) was used for color names and hue numbers. Cultures were observed after incubation at 27°C for 2 weeks.

**Table 1**

| Cultural characteristics of strain K93-0711 | | | | |
|---|---|---|---|---|
| Medium | Growth | Reverse color | Aerial mass color | Soluble pigment |
| Sucrose-nitrate agar** | Moderate, ivory tint (2cb) | Ivory tint(2cb) | Poor, pearl (2ba) | None |
| Glucose-asparagine agar | Moderate, pearl pink(3ca) | Pearl pink (3ca) | Poor, pearl (2ba) | None |
| Glycerol-asparagine agar* | Good, natural(2dc) | Natural (2dc) | Abundant, silver gray (3fe) | None |
| Inorganic salts-starch agar* | Moderate, bamboo(2gc) | Lt. mustard tan(2ie) | Moderate, covert gray (2fe) | None |
| Tyrosine agar* | Moderate, lt.brown(3lg) | Lt. brown(3lg) | Moderate, natural(2dc) | Lt. brown (3lg) |
| Oatmeal agar* | Good, bamboo(2gc) | Bamboo (2gc) | Moderate, gray(2fe) | None |
| Yeast extract-malt extract agar* | Good, It. mustard tan(2ie) | Biscuit (2ec) | Abundant. covert gray (2fe) | None |
| Nutrient agar** | Moderate, putty(1½ ec) | Ivory (2db) | Moderate, alabaster tint~ pearl gray (13ba ~ 13dc) | None |
| Peptone-yeast extract-iron agar** | Moderate, It. mustard tan(2ie) | Lt. mustard tan(2ie) | None | Lt. brown (3lg) |
| Glucose-nitrate agar** | Moderate, biscuit (2ec) | Bamboo (2gc) | Moderate, alabaster tint(13ba) | None |
| Glycerolcalcium malate agar** | Good, covert tan (2ge) | Bamboo (2gc) | Abundant, alabaster tint~gray (13ba ~i) | None |
| Glucosepeptone agar** | Good, mustard tan (1 ½ lc) | Golden brown(3pg) | Abundant, alabaster tint(13ba) | None |

| | | | | |
|---|---|---|---|---|
| * Medium recommended by International Streptomyces Project. | | | | |
| ** Medium recommended by S. A. Waksman. | | | | |

### III. Physiological properties:

| | | | |
|---|---|---|---|
| (1) | Melanin formation | | |
| | (a) | Tyrosine agar | - |
| | (b) | Peptone-yeast extract iron agar | - |
| | (c) | Tryptone-yeast extract broth | - |
| (2) | Tyrosinase reaction | | - |
| (3) | H₂S production | | - |
| (4) | Nitrate reduction | | + |
| (5) | Liquefaction of gelatin (21-23 °C) (glucose-peptone-gelatin agar) | | + |
| (6) | Hydrolysis of starch | | + |
| (7) | Coagulation of milk (37 °C) | | - |
| (8) | Peptonization of milk (37 °C) | | + |
| (9) | Temperature range for growth | | 15 - 39 °C |
| (10) | Utilization of carbon sources (Pridham and Gottlieb's agar medium) utilized: glucose, arabinose, xylose and rhamnose | | |
| (11) | Cellulolytic activity | | - |

### IV. Chemical composition:

The DAP isomer in whole-cell of the strain is LL-type.

The taxonomic properties of the present strain K93-0711 are summearized as follows. DAP isomer in whole-cell is LL-type. Vegetative mycelia grew abundantly on various agar media, and show bacillary elements. The aerial mycelia are straight with forming long spore chains. Surface of spores is smooth. The vegetative mycelia show a brown color on various media and mass color of aerial mycelia shows white to gray. Soluble pigment formation is slightly brownish in Tyrosine agar and peptone-yeast extract iron agar.

Based on the taxonomic properties described above, strain K93-0711 is considered to belong the genus Streptomyces. The strain was referred to as Streptomyces sp. K93-0711, and was deposited in the National Institute of Bioscience and Human Technology Agency of Industrial Science and Technology, Japan, under the name Streptomyces sp. K93-0711, and the accession No. is FERM P-15253. The strain was transferred to international deposit of microorganisms based on Budapest Treaty on the International Recognition of the Deposit of Microorganisms for the Purposes of Patent Procedure on December 4, 1996 as accession No. FERM BP-5764. Also. Depositor is The Kitasato Institute of 9 - 1, Shirokane 5-chome, Minato-ku, Tokyo.

Preferable example of the strain of the present invention for production of the bioactive substance K93-0711 I-1 and I-2 was explained hereinabove. However Streptomyces are known to easily mutate by using, for example, ultraviolet, X-ray irradiation or mutagens such as N-methyl-N-nitro-N-nitrosoguanidine or ethylmethanesulfonate. Accordingly, these artificial mutants and natural mutants involving Streptomyces sp. K93-0711 and substance K93-0711 I-1 and I-2 producing strains belonging to genus Streptomyces can be used in the present invention.

In the present invention, K93-0711 I-1 and I-2 producing microorganisms belonging to genus Streptomyces are cultured in the preferable medium. Culture of the strain can be made by using usual medium for Streptomyces. Nutrient medium containing assimilable carbon sources and nitrogen sources and if required inorganic salts for microorganisms can be used.

Examples of the assimilable carbon sources are glucose. molasses,starch, dextrin, corn steep liquor, glycerol and organic acid in combination or alone. Examples of assimilable nitrogen sources are commercially available organic nitrogen sources such as peptone, meat extract, yeast extract, dry yeast, soybean powder, conrn steep liquor, cotton seed powder, caseine, soybean protein hydrolysates, amino acids and urea, and inorganic nitrogen sources such as nitrate and ammonium salts, in combination or alone.

Further, if necessary, inorganic salt such as sodium, potassium. calcium, magnesium and phosphate can be used. Trace nutrient elements, growth promotors and precursors for K93-0711 I-1 ane I-2 production can be added.

Cultivation can proceeded in aerobic shake culture or aeration culture under aerobic condition. Industrial production can preferably be made under submerged aeration culture. The pH of the medium is preferably neutral. Culture temperature is at 20 - 37°C, preferably 24 - 30 °C, and most preferably at 27 °C. Culturing time is, usually for 3 - 6 days in liquid culture, and can be terminated when maximum production of the substance K93-0711 I-1 and I-2 is observed. These culture conditions such as culture medium composition, pH. temperature, stirring and aeration can be adjusted for preferable production using microbial strain and other conditions. In the liquid culture, anti-foam agent such as silicon oil. vegetable oil and surface active agents can be added.

The thus produced bioactive substance is accumulated in culture filtrate or cultured mycelia. Cultured mass was filtered by adding filter aid such as Celite or Hyflosupercel, or centrifuged to separate culture filtrate and mycelia. Organic solvent extracts of culture filtrate and mycelai were combined and concentrated, then K93-0711 I-1 and I-2 can be isolated from the extracts.

Cultured mass can directly be extracted without filtration with water immiscible organic solvent. Extraction of the substance can be made from culture filtrate or mycelia if the substance is contained partially in each one.

For purification of K93-0711 I-1 and I-2 from cultured mass, organic solvent extract of culture filtrate or mycelia (in combination on separately) is concentrated, then extracted further with) a water immiscibleorganic solvent such as ethyl acetate or chloroform to transfer K93-0711 I-1 and I-2 in to the organic solvent.

The thus obtained organic solvent extracted layer is. if require dried by adding drying agents such as anhydrous sodium sulfate and beads gel, and is distilled in vacuo. In this concentrating operation, though the substance K93-0711 I-1 and I-2 is quite stable, the process is preferably proceeded below 50 °C. The substance K93-0711 I-1 and I-2 can be precipitated by adding an organic solvent such as hexane or petroleum ether to the residue.

The precipitate is washed several times with hexane, and the crude substance K93-0711 I-1 and I-2 can be obtained by vacuum suction or centrifugation. Further purification can be made by various procedures such as utilising with a difference in solubilities of the substance K93-0711 I-1 and I-2 and other mixed impurities, a difference in distribution to the two liquid phases and a difference in absorption to the absorbents carrier. Chromatography is the most preferable purification means.

Preferable chromatographic procedures for purification of the substance K93-0711 I-1 and I-2 are absorption chromatography using silica gel, alumina, activated charcoal cellulose, adsorption resin such as hydroxyappatite HP-20, reverse phase distribution chromatography using silanizing silica gel or octadecyl silanizing silica gel, gel filtration chromatography using a molecular sieve such as Sephadex LH-20 or Toyopearl ( TM, Toso Corp. ), and ion exchange chromatography.

The substance K93-0711 I-1 and I-2 can be purified by using these procedures, for example chromatography, electrophoresis, counter current distribution and ultrafiltration, in combination or alone, or in any arrangement, or repeatedly. One embodiment is, for example, that the crude substance is dissolved in small amount of chloroform, adsorbed in silica gel, and subjected to chromatography using mixture of chloroformacetone. The active fraction is concentrated in vacuo, and the concentrate is dissolved in small amount of methanol. The solution is treated by gel filtration chromatography eluted with methanol to purify the substance K93-0711 I-1 and I-2.

Physico-chemical and biological properties of the substance K93-0711 I-1 and I-2 are shown in the following. Physico-chemical properties:

### Bioactive substance K93-0711 I-1

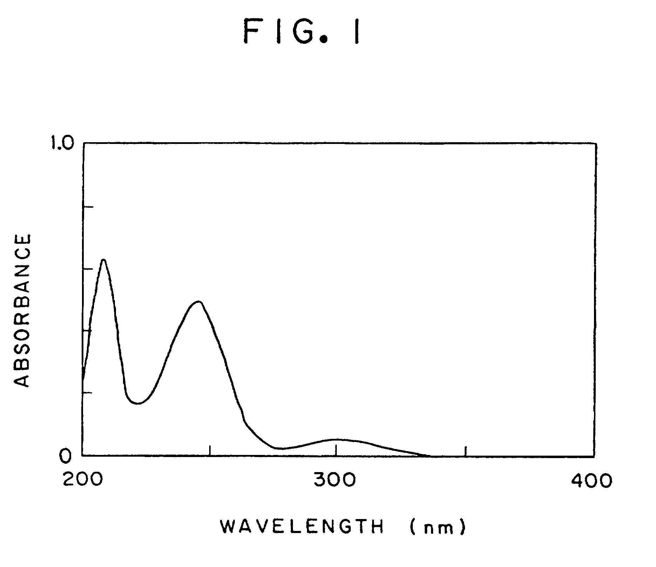

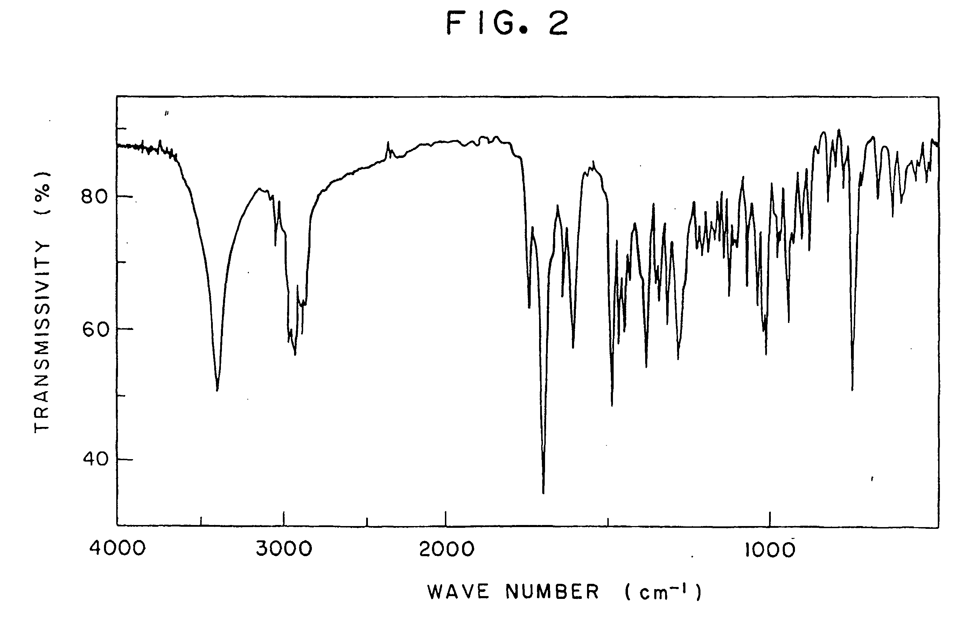

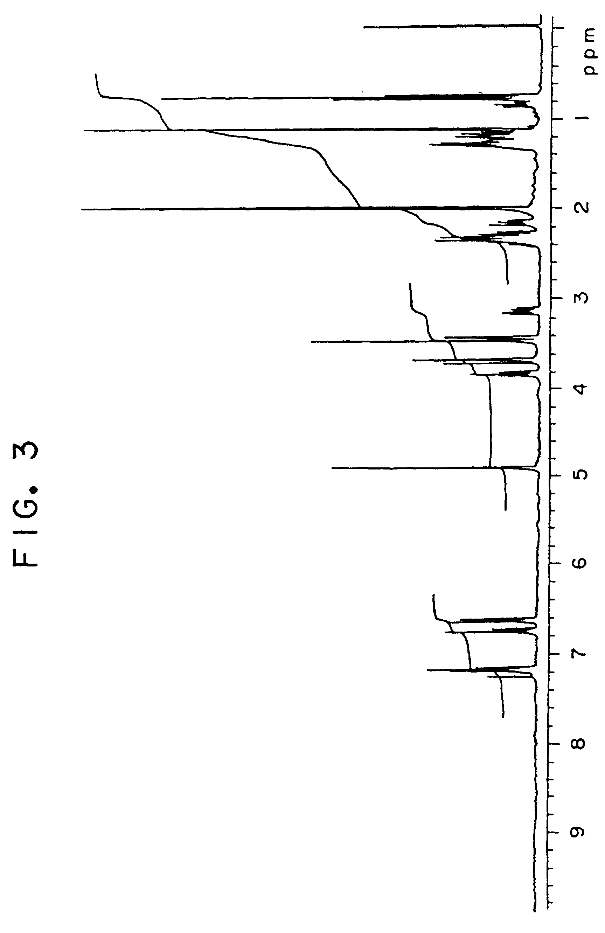

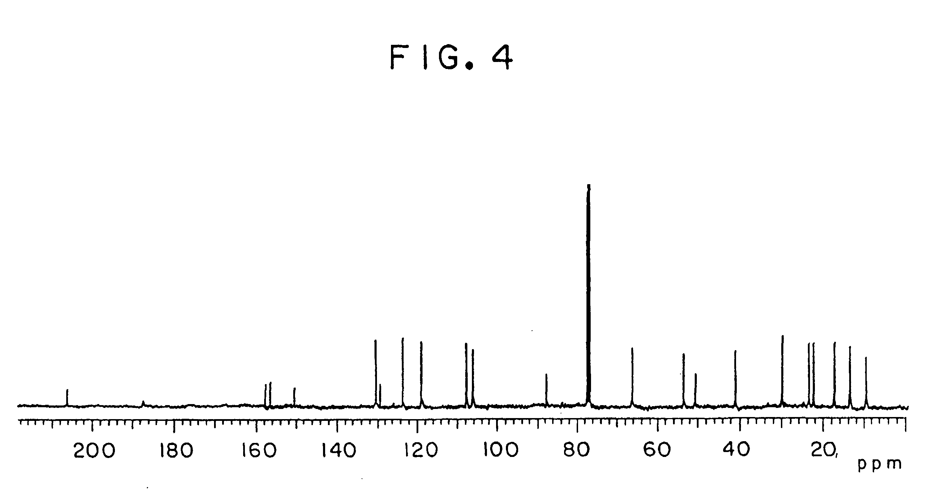

(1) Nature: pale yellow oily substance
(2) Molecular formula: C₂₂H₂₇O₄N ( high resolution FAB mass spectrum )
(3) Specific rotation [α]²⁴ _{D} +44.4° ( c. 0.3, MeOH )
(4) Molecular weight: 369 ( FAB mass spectrum )
(5) Ultraviolet and visible light absorption spectrum: UV spectrum in methanol is shown,in Fig. 1 with specific absorption maximum at 299 (log ε 3.28), 245 ( log ε 4.26 ) and 207 (log ε 4.38 ) nm
(6) Infrared absorption spectrum: IR spectrum in KBr is shown in Fig. 2 with absorption bands at 3390, 2927, 1741, 1695, 1603, 1487, 1381, 1281 and 756 cm⁻¹
(7) Solubility: Soluble in acetone, ethyl acetate, ethyl ether, hexane, methanol, ethanol, chlroform and benzene Slightly soluble in water
(8) Color reaction: positive for sulfuric acid, Ehrlich and iodine.
(9) NMR spectrum: ¹H-NMR spectrum (in deuterated chloroform, 400 MHz) and ¹³C-NMR spectrum ( in deuterated chloroform, 100.58 MHz )
   ( Varian Japan Corp., XL-400 NMR Spectrometer ) are shown in Fig.3 and Fig. 4. Chemical shifts of ¹H and ¹³C are shown in Table 2.

**Table 2**

| ¹³C - shift | ¹H - shift ( J ) |
|---|---|
| 206.4 ( s ) | |
| 206.3 ( s ) | |
| 157.7 ( s ) | |
| 156.6 ( s ) | |
| 150.5 ( s ) | |
| 130.5 ( d ) | 7.19 ( 1H, m ) |
| 129.5 ( s ) | |
| 123.5 ( d ) | 7.19 ( 1H, m ) |
| 119.1 ( d ) | 6.75 ( 1H, td, 7.3. 0.5 ) |
| 108.0 ( d ) | 6.63 ( 1H, d, 8.0 ) |
| 106.2 ( d ) | 4.93 ( 1H, s ) |
| 88.0 ( s ) | |
| 66.6 ( t ) | 3.84 ( 1H, m) 3.14 ( 1H, m ) |
| 53.7 ( t ) | 3.69 ( 1H, d, 14.2 ) |
| | 3.45 ( 1H, d, 14.2 ) |
| 50.6 ( s ) | |
| 41.1 ( t ) | 2.33 ( 1H, dd, 12.0, 9.0 ) |
| | 2.17 ( 1H, ddd, 12.0, 5.0, 1.5 ) |
| 29.9 ( t ) | 1.29 ( 2H, m ), 1.29 |
| 23.5 ( t ) | 2.38 ( 1H, m) 2.36 ( 1H, m) |
| 22.7 ( t ) | 1.19 ( 2H, m) |
| 17.3 ( q ) | 1.12 ( 3H, s) |
| 13.8 ( q ) | 0.76 ( 3H, t, 7.0 ) |
| 9.4 ( q ) | 2.00 ( 3H, s ) |

### Bioactive substance K93-0711 I-2

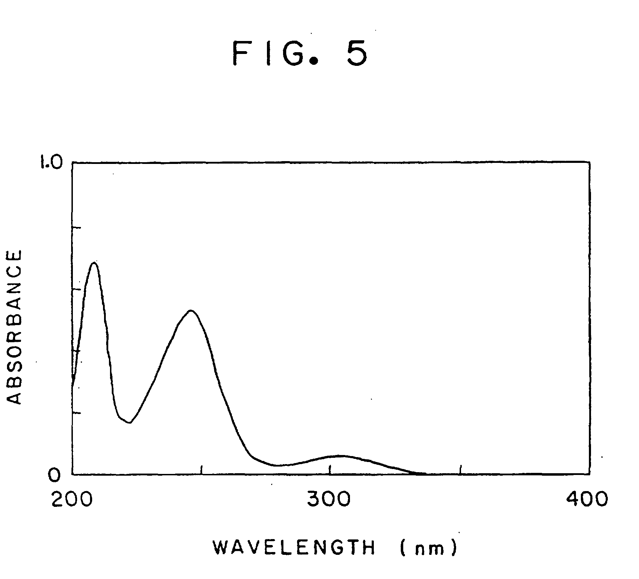

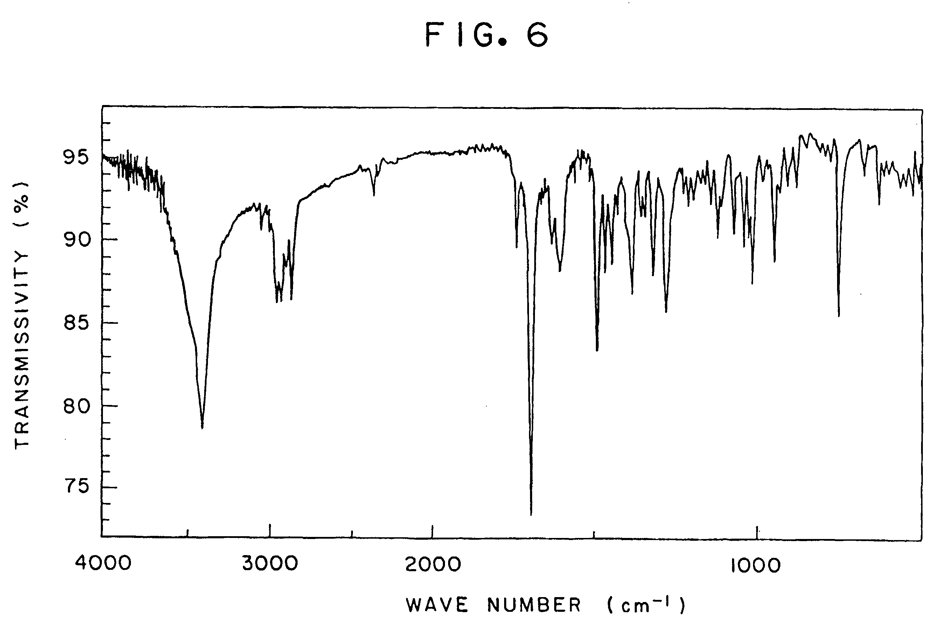

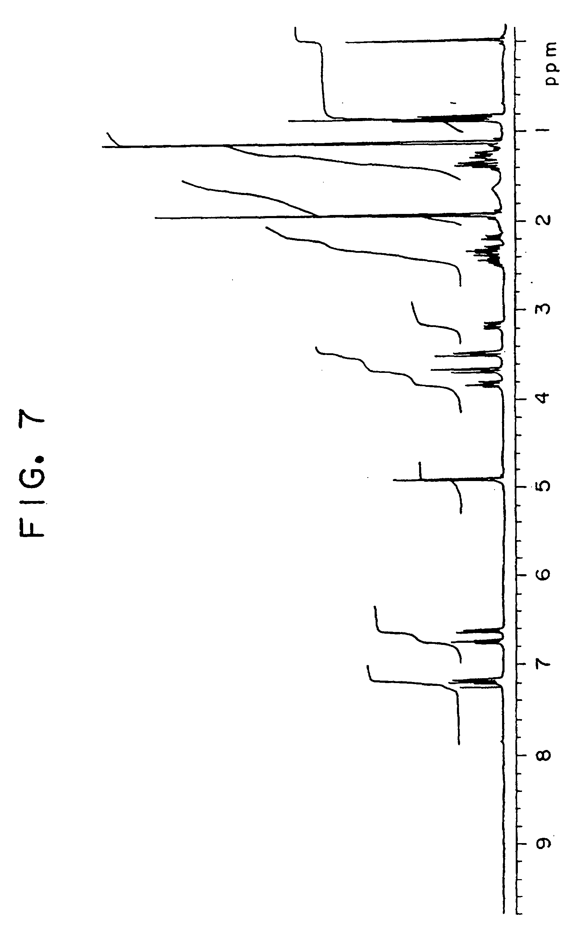

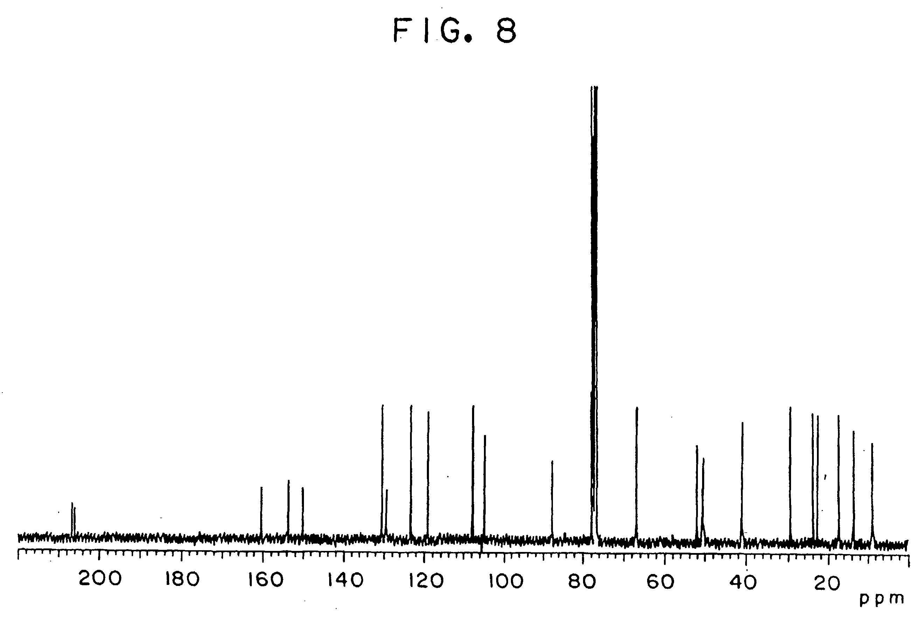

(1) Nature: pale yellow oily substance
(2) Molecular formula: C₂₂H₂₇O₄N ( high resolution FAB mass spectrum )
(3) Specific rotation [α]²⁴_{D} +25.6° ( c. 0.3, MeOH )
(4) Molecular weight: 369 ( FAB mass spectrum )
(5) Ultraviolet and visible light absorption spectrum: UV spectrumin methanol is shown in Fig. 5 with specific absorption maximum at 208 ( log ε 4.40 ), 246 ( log ε 4.29 ) and 304 ( log ε 3.28 ) nm
(6) Infrared absorption spectrum: IR spectrum in KBr is shown in Fig. 6 with absorption bands at 3410, 2927, 1738, 1693, 1597, 1489. 1380. 1282 and 756 cm⁻¹
(7) Solubility: Soluble in acetone, ethyl acetate, ethyl ether. hexane, methanol, ethanol, chlroform and benzene Slightly soluble in water
(8) Color reaction: positive for sulfuric acid. Ehrlich and iodine.
(9) NMR spectrum: ¹H-NMR spectrum (in deuterated chloroform, 400 MHz) and ¹³C-NMR spectrum ( in deuterated chloroform, 100.58 MHz )
   (Varian Japan Corp., XL-400 NMR Spectrometer) are shown in Fig. 7 and Fig. 8 . Chemical shifts of ¹H and ¹³C are shown in Table 3.

**Table 3**

| ¹³C - shift | ¹H - shift |
|---|---|
| 206.6 ( s ) | |
| 205. 9 ( s ) | |
| 160.3 ( s) | |
| 153.7 ( s ) | |
| 150.4 ( s) | |
| 130.5 ( d ) | 7.19 ( 1H, m ) |
| 129.4 ( s ) | |
| 123.5 ( d ) | 7.19 ( 1H, m ) |
| 118.9 ( d ) | 6.74 ( 1H, td, 7.2, 1.0 ) |
| 107.8 ( d ) | 6.62 ( 1H, d, 8.0 ) |
| 105.0 ( d ) | 4.92 ( 1H, s ) |
| 87.9 ( s ) | |
| 66.8 ( t ) | 3.85 ( 1H, ddd, 12.0, 7.9, 1.5 ) |
| | 3.17 ( 1H, ddd, 12.0, 9.2, 5.0 ) |
| 52.1 ( t ) | 3.69 ( 1H, d. 14.9 ) |
| | 3.49 ( 1H, d, 14.9 ) |
| 50.5 (s) | |
| 41.2 ( t ) | 2.33 ( 1H, ddd, 12.0, 7.9 , 1.5 ) |
| | 2.19 ( 1H, ddd, 12.0, 9.2, 1.5 ) |
| 29.5 ( t ) | 1.39 ( 2H, m ) |
| 24.0 ( t ) | 2.46 ( 1H, m ) 2.37 ( 1H, m ) |
| 22.9 ( t ) | 1.25 ( 2H. m ) |
| 17.5 ( q ) | 1.12 ( 3H, s ) |
| 13.8 ( q ) | 0.86 ( 3H, t, 7.1 ) |
| 9.1 ( q ) | 1.94 ( 3H, s ) |

### Biological properties:

### Evaluation of IL-6 activity modified action by MH-60 cells

MH-60. BSF2 cells ( hereinafter disignated as MH-60) derived from mouse bone marrow having IL-6 dependent growth acivity was used. IL-6 modification activity was examined using MH-60 by indicating cell growth activity against 0.2 u/ml IL-6. MH-60 cells 5 × 10³/100*µ*l suspended in RPM1 1640 medium containing 10% fetal bovine serum ( FBS ) were plated in 96 well micro-plate. The bioactive substance K93-0711 I-1 and I-2 5 *µ*l was added, and 0.2 u/ml IL-6 solution 100 *µ*l was added in every well, then incubated at 37 °C for 72 hours.

Cells were stained by MTT method [3-(4, 5-dimethylthiazol-2-yl ) -2, 5-diphenyl-2H-tetrazolium bromide, then colorimetrically measured at492 nm and at 630 nm ( reference ) to calculate cell growth rate. Simultaneously, cell growth rate of IL-6-induced growth of IL-6-independent MH 60 cells established by cloning was measured. Results are shown in Table 4 and Table 5.

**Table 4**

| Inhibitory K93-0711 I-1 on growth of MH-60 cells | | |
|---|---|---|
| Concentration of K93-0711 I-1 (µg/ml) | Growth rate of IL-6 dependent MH-60 cells (%) | Growth rate of IL-6 independent MH-60 cells (%) |
| 25.0 | 5.25 | 102.5 |
| 12.5 | 19.85 | 101.2 |
| 6.25 | 35.2 | 97.5 |
| 3.13 | 51.1 | 96.5 |
| 1.56 | 82.1 | 92.3 |
| 0.78 | 88.3 | 100.8 |

**Table 5**

| Inhibitory K93-0711 1-2 on growth MH-60 cells | | |
|---|---|---|
| Concentration of K93-0711 I-2 ( *µ*g/ml ) | Growth rate of IL-6 dependent MH-60 cells ( % ) | Growth rate of IL-6 independent MH-60 cells ( % ) |
| 25.0 | 17.3 | 103.5 |
| 12.5 | 46.3 | 87.8 |
| 6.25 | 70.0 | 99.7 |
| 3.13 | 84.1 | 97.5 |
| 1.56 | 94.3 | 97.8 |
| 0.78 | 91.7 | 102.5 |

### EFFECT OF THE INVENTION

As clearly shown in Table 4 and 5, the bioactive substance K93-0711 I-1 and I-2 of the present invention selectively suppressed IL-6 activity. Therefore, the substance is effective for treatment of diseases involving IL-6, for example improvement for cancer cachexia, multiple myeloma and rheumatoid arthritis.

### EXAMPLE

The following example illustrates the present invention but is not construed as limiting.

A culture of Streptomyces sp. K93-0711 ( FERM BP-5764 ) from agarslant consisting of 1.0% starch, 0.3% NZ amine, 0.1% yeast extract. 0.1%meat extract, 0.3% calcium carbonate and 1.0% agar, cultured at 27°C for6 days, was inoculated into test tubes (φ2 × 20 cm ) containing 10 ml of a medium consisting of 0.1% glucose, 2.4% starch, 0.3% peptone. 0.5% yeast extract, 0.3% meat extract, and 0.4% calcium carbonate ( pH 7.0 ).The test tube was incubated on a rotary shaker at 200 rpm at 27°C for 72 hours to prepare seed culture. The seed culture ( 2 ml, 2% ) was inoculated into 500-ml Erlenmeyer flask containing 100 ml of the same medium ( pH 7.0 ), and fermented under the same culture conditions. Then 700 ml ( 1% ) of the seed culture was transferred into a 100-liter jar fermenter containing 70 liters of a medium consisting of 2.4% starch, 0.1% glucose, 0.3% peptone, 0.5% yeast extract, 0.3% meat extarct, 0.4% calcium carbonate, 0.5% Allophosite ( 100 mesh ) and trace metals solution. The fermentation was carried out at 27°C for 4 days with agitation and aeration to obtain cultured mass approximately 70 liters.Ethyl acetate 40 liter was added into the cultured mass and stirred well, then centrifuged to separate ethyl acetate layer.

The ethyl acetate extract was concentrated using rotary evaporator to obtain crude extract 11.5 g. The crude extract was applied to silicagel column chromatography ( E. Merck, 70-230 mesh ), inner diameter 80 mm, length 260 mm column, packed with chloroform. The silica gel columnwas washed with chloroform 1 liter. The materials were eluted with a mixed solvent of chloroform-methanol (100 : 1) to obtain active fraction. The fractions were concentrated in vacuo yield an oily material 561.4 mg. The substance was dissolved in small amountof methanol and the solution was applied to HPLC ( Senshu pack pegacil, 20 × 250 mm, solvent 50% acetonitrile-H₂O ) to obtain the substance K93- 0711 I-1, 11.8 mg and K93-0711 I-2, 20.8 mg.

The substance K93-0711 I-1 and I-2 were assumed to be stereoisomers because of the same UV absorption, molecular weight and molecular formula.

### References:

1. Gideon Strassmann et al., "Mechanism of Experimental Cancer Cachexia,Local Involvement of IL-1 in Colon - 26 Tumor", J. Immunol. 150: 2341- 2345, 1993.
2. Gideon Strassmann et al., "Evidence for the Involvement of Interleukin 6 in Experimental Cancer Cachexia", J. Clin. Invest. 89: 1681-1684. 1992.
3. R.S. Kerbel, "Expression of Multi-cytokine Resistance and Multi Growth Factor Independence in Advanced Stage Metastatic Cancer", Am. J. Pathol. 141, 519-524, 1992.
4. Gideon Strassmann et al., "Mechanism of Experimental Cancer Cachexia, Interaction between Mononuclear Phagocytes and Colon-26 Carcinoma and Its Relevance to IL-6-Mediated Cancer Cachexia", J. Immunol. 148, 3674- 3678, 1992.

### BRIEF EXPLANATION OF THE DRAWINGS

Fig. 1 : UV absorption spectrum of the bioactive substance K93-0711 I-1 ( in methanol )

Fig. 2 : IR absorption spectrum of the bioactive substance K93-0711 I-1 ( KBr )

Fig. 3 : ¹H-NMR spectrum ( in deutrium chloroform, 400 MHz ) of K93-0711 I-1

Fig. 4 : ¹³C-NMR spectrum ( in deutrium chloroform, 100.58 MHz ) of K93-0711 I-1

Fig. 5 : UV absorption spectrum of K93-0711 I-2 ( in methanol )

Fig. 6 : IR absorption spectrum of the bioactive substance K93-0711 I-2 ( KBr )

Fig. 7 : ¹H-NMR spectrum ( in deutrium chloroform, 400 MHz ) of K93-0711 I-2

Fig. 8 : ¹³C-NMR spectrum ( in deutrium chloroform, 100.58 MHz ) of K93-0711 I-2

## Claims

1. A bioactive substance which is denoted as K93-0711 I-1 or K93-0711 I-2, which is obtainable by culturing *Streptomyces* sp. K93-0711 (FERM BP-5764) and which has the following physicochemical properties:
(1) Bioactive substance K93-0711 I-1;
(i) Molecular formula : C₂₂H₂₇O₄N (high resolution FAB mass spectrum)
(ii) Molecular weight : 369 (FAB mass spectrum)
(iii) Ultraviolet and visible light absorption spectrum : UV spectrum in methanol has specific absorption maxima at 299 (log ε 3.28), 245 (log ε 4.26) and 207 (log **ε** 4.38) nm
(iv) Infrared absorption spectrum:
IR spectrum in KBr has absorption bands at 3390, 2927, 1741, 1695, 1603, 1487, 1381, 1281 and 756 cm⁻¹
(v) Specific rotation [α]²⁴_{D} +44.4° (c. 0.3, MeOH)
(vi) Solubility:
Soluble in acetone, ethyl acetate, ethyl ether, hexane, methanol, ethanol, chloroform and benzene
Slightly soluble in water
(vii) ¹H-NMR spectrum (in deuterated chloroform, 400 MHz and ¹³C-NMR spectrum (in deuterated chloroform, 100.58 MHz)
| ¹³C - shift | ¹H shift(J) |
|---|---|
| 206.4 (s) | |
| 206.3 (s) | |
| 157.7 (s) | |
| 156.6 (s) | |
| 150.5 (s) | |
| ¹³ C - shift | ¹H shift(J) |
|---|---|
| 130.5 (d) | 7.19 (1H, m) |
| 129.5 (s) | |
| 123.5 (d) | 7.19 (1H, m) |
| 119.1 (d) | 6.75 (1H, td, 7.3, 0.5) |
| 108.0 (d) | 6.63 (1H, d, 8.0) |
| 106.2 (d) | 4.93 (1H, s) |
| 88.0 (s) | |
| 66.6 (t) | 3.84 (1H, m) 3.14 (1H, m) |
| 53.7 (t) | 3.69 (1H, d, 14.2) |
| | 3.45 (1H, d, 14.2) |
| 50.6 (s) | |
| 41.1 (t) | 2.33 (1H, dd, 12.0, 9.0) |
| | 2.17 (1H, ddd, 12.0, 5.0, 1.5) |
| 29.9 (t) | 1.29 (2H, m) 1.29 |
| 23.5 (t) | 2.38 (1H, m) 2.36 (1H, m) |
| 22.7 (t) | 1.19 (2H, m) |
| 17.3 (q) | 1.12 (3H, s) |
| 13.8 (q) | 0.76 (3H, t, 7.0) |
| 9.4 (q) | 2.00 (3H, s) |
(viii) Color reaction : positive for sulfuric acid, Ehrlich and iodine.
(2) Bioactive substance K93-0711 I-2;
(i) Molecular formula : C₂₂H₂₇O₄N (high resolution FAB mass spectrum)
(ii) Molecular weight : 369 (FAB mass spectrum)
(iii) Ultraviolet and visible light absorption spectrum: UV spectrum in methanol has specific absorption maxima at 304 (log ε 3.28), 246 (log ε 4.29) and 208 (log ε 4.40) nm
(iv) Infrared absorption spectrum
IR spectrum in Kbr has absorption bands at 3410, 2927, 1738, 1693, 1597, 1489, 1380, 1282 and 756 cm ⁻¹
(v) Specific rotation [α]²⁴_{D} +25.6° (c. 0.3, MeOH)
(vi) Solubility :
Soluble in acetone, ethyl acetate, ethyl ether, hexane, methanol, ethanol, chloroform and benzene
Slightly soluble in water
(vii) ¹H-NMR spectrum (in deuterated chloroform, 400 MHz) and ¹³C-NMR spectrum (in deuterated chloroform, 100.58 MHz)
| ¹³C - shift | ¹H - shift |
|---|---|
| 206.6 (s) | |
| 205.9 (s) | |
| 160.3 (s) | |
| 153.7 (s) | |
| 150.4 (s) | |
| 130.5 (d) | 7.19 (1H, m) |
| 129.4 (s) | |
| 123.5 (d) | 7.19 (1H, m) |
| 118.9 (d) | 6.74 (1H, td, 7.2, 1.0) |
| 107.8 (d) | 6.62 (1H, d, 8.0) |
| 105.0 (d) | 4.92 (1H, s) |
| 87.9 (s) | |
| 66.8 (t) | 3.85 (1H, ddd, 12.0, 7.9, 1.5) |
| | 3.17 (1H, ddd, 12.0, 9.2, 5.0) |
| 52.1 (t) | 3.69 (1H, d, 14.9) |
| | 3.49 (1H, d, 14.9) |
| ¹³C - shift | ¹H - shift |
|---|---|
| 50.5 (s) | |
| 41.2 (t) | 2.33 (1H, ddd, 12.0, 7.9, 1.5) |
| | 2.19 (1H, ddd, 12.0, 9.2, 1.5) |
| 29.5 (t) | 1.39 (2H, m) |
| 24.0 (t) | 2.46 (1H, m) 2.37 (1H, m) |
| 22.9 (t) | 1.25 (2H, m) |
| 17.5 (q) | 1.12 (3H, s) |
| 13.8 (q) | 0.86 (3H, t, 7.1) |
| 9.1 (q) | 1.94 (3H, s) |
(viii) Color reaction : positive for sulfuric acid, Ehrlich and iodine.

2. A process for production of bioactive substance K93-0711 I-1 or I-2 as defined in claim 1, comprising culturing *Streptomyces* sp. K93-0711 (FERM BP-5764), or a mutant thereof which produces said bioactive substance K93-0711 I-1 or I-2, in a medium to accumulate said bioactive substance K93-0711 I-1 or I-2 in the medium; and isolating said bioactive substance therefrom.

3. *Streptomyces* sp. K93-0711 (FEBM BP-5764), or a mutant thereof which produces bioactive substance K93-0711 I-1 or I-2 as defined in claim 1.

4. A bioactive substance as defined in claim 1 for use in a method of treatment of the human or animal body by therapy.

5. A bioactive substance as claimed in claim 4 for use in the treatment of a disease involving IL-6.

6. A bioactive substance as claimed in claim 4 for use in the treatment of cancer cachexia, multiple myeloma or rheumatoid arthritis.

7. A pharmaceutical composition comprising a bioactive substance as defined in claim 1 and a pharmaceutically acceptable carrier or diluent.

## Patentansprüche

1. Bioaktiver Stoff, welcher als K93-0711 I-1 oder K93-0711 I-2 bezeichnet ist, welcher durch Kultivieren von *Streptomyces sp.* K93-0711 (FERM BP-5764) erhältlich ist und welcher die folgenden phyikochemischen Eigenschaften hat:
(1) Bioaktiver Stoff K93-0711 I-1;
(i) Molekularformel: C₂₂H₂₇O₄N (hochauflösendes FAB-Massenspektrum)
(ii) Molekulargewicht: 369 (FAB Massenspektrum)
(iii) Ultraviolett- und sichtbares Licht-Absorptionsspektrum: UV-Spektrum in Methanol hat spezifische Absorptionsmaxima bei 299 (log ε 3,28), 245 (log ε 4,26) und 207 (log ε 4,38) nm
(iv) Infrarot-Absorptionsspektrum:
IR-Spektrum in KBr hat Absorptionsbanden bei 3390, 2927, 1741, 1695, 1603, 1487, 1381, 1281 und 756 cm⁻¹
(v) Spezifische Drehung [α]²⁴_{D} +44,4° (c. 0,3, MeOH)
(vi) Löslichkeit:
Löslich in Aceton, Ethylacetat, Ethylether, Hexan, Methanol, Ethanol, Chloroform und Benzol
Schwach löslich in Wasser
(vii) ¹H-NMR-Spektrum (in deuteriertem Chloroform, 400 MHz) und ¹³C-NMR-Spektrum (in deuteriertem Chloroform, 100,58 MHz)
| ¹³C-Verschiebung | ¹H-Verschiebung (J) |
|---|---|
| 206,4 (s) | |
| 206,3 (s) | |
| 157,7 (s) | |
| 156,6 (s) | |
| 150,5 (s) | |
| ¹³C-Verschiebung | ¹H-Verschiebung (J) |
|---|---|
| 130,5 (d) | 7,19 (1H, m) |
| 129,5 (s) | |
| 123,5 (d) | 7,19 (1H, m) |
| 119,1 (d) | 6,75 (1H, td, 7,3, 0,5) |
| 108,0 (d) | 6,63 (1H, d, 8,0) |
| 106,2 (d) | 4,93 (1H, s) |
| 88,0 (s) | |
| 66,6 (t) | 3,84 (1H, m) 3,14 (1H, m) |
| 53,7 (t) | 3,69 (1H, d, 14,2) |
| | 3,45 (1H, d, 14,2) |
| 50,6 (s) | |
| 41,1 (t) | 2,33 (1H, dd, 12,0, 9,0) |
| | 2,17 (1H, ddd, 12,0, 5,0, 1,5) |
| 29,9 (t) | 1,29 (2H, m) 1,29 |
| 23,5 (t) | 2,38 (1H, m) 2,36 (1H, m) |
| 22,7 (t) | 1,19 (2H, m) |
| 17,3 (q) | 1,12 (3H, s) |
| 13,8 (q) | 0,76 (3H, t, 7,0) |
| 9,4 (q) | 2,00 (3H, s) |
(viii) Farbreaktion: Positiv für Schwefelsäure, Ehrlich und Jod.
(2) Bioaktiver Stoff K93-0711 I-2;
(i) Molekularformel: C₂₂H₂₇O₄N (hochauflösendes FAB-Massenspektrum)
(ii) Molekulargewicht: 369 (FAB Massenspektrum)
(iii) Ultraviolett und sichtbares Licht-Absorptionsspektrum: UV-Spektrum in Methanol hat spezifische Absorptionsmaxima bei 304 (log ε 3,28), 246 (log ε 4,29) und 208 (log ε 4,40) nm
(iv) Infrarot-Absorptionsspektrum:
IR-Spektrum in KBr hat Absorptionsbanden bei 3410, 2927, 1738, 1693, 1597, 1489, 1380, 1282 and 756 cm⁻¹
(v) Spezifische Drehung [α]²⁴_{D} +25,6° (c. 0,3, MeOH)
(vi) Löslichkeit:
Löslich in Aceton, Ethylacetat, Ethylether, Hexan, Methanol, Ethanol, Chloroform und Benzol
Schwach löslich in Wasser
(vii) ¹H-NMR-Spektrum (in deuteriertem Chloroform, 400 MHz) und ¹³C-NMR-Spektrum (in deuteriertem Chloroform, 100,58 MHz)
| ¹³C-Verschiebung | ¹H-Verschiebung |
|---|---|
| 206,6 (s) | |
| 205,9 (s) | |
| 160,3 (s) | |
| 153,7 (s) | |
| 150,4 (s) | |
| 130,5 (d) | 7,19 (1H, m) |
| 129,4 (s) | |
| 123,5 (d) | 7,19 (1H, m) |
| 118,9 (d) | 6,74 (1H, td, 7,2, 1,0) |
| 107,8 (d) | 6,62 (1H, d, 8,0) |
| 105,0 (d) | 4,92 (1H, s) |
| 87,9 (s) | |
| 66,8 (t) | 3,85 (1H, ddd, 12,0, 7,9, 1,5) |
| | 3,17 (1H, ddd, 12,0, 9,2, 5,0) |
| 52,1 (t) | 3,69 (1H, d, 14,9) |
| | 3,49 (1H, d, 14,9) |
| 50,5 (s) | |
| 41,2 (t) | 2,33 (1H, ddd, 12,0, 7,9, 1,5) |
| | 2,19 (1H, ddd, 12,0, 9,2, 1,5) |
| 29,5 (t) | 1,39 (2H, m) |
| 24,0 (t) | 2,46 (1H, m) 2,37 (1H, m) |
| 22,9 (t) | 1,25 (2H, m) |
| 17,5 (q) | 1,12 (3H, s) |
| 13,8 (q) | 0,86 (3H, t, 7,1) |
| 9,1 (q) | 1,94 (3H, s) |
(viii) Farbreaktion: Positiv fiir Schwefelsäure, Ehrlich und Jod.

2. Verfahren zur Herstellung von bioaktivem Stoff K93-0711 I-1 oder I-2, wie in Anspruch 1 definiert, das umfaßt:
Kultivieren von *Streptomyces sp.* K93-0711 (FERM BP-5764) oder einer Mutanten davon, welche den bioaktiven Stoff K93-0711 I-1 oder I-2 herstellt, in einem Medium, um den bioaktiven Stoff K93-0711 I-1 oder I-2 in dem Medium anzureichern, und Isolieren des bioaktiven Stoffs daraus.

3. *Streptomyces sp.* K93-0711 (FERM BP-5764) oder eine Mutante davon, welche den bioaktiven Stoff K93-0711 I-1 oder I-2, wie in Anspruch 1 definiert, herstellt.

4. Bioaktiver Stoff, wie in Anspruch 1 definiert, zur Verwendung in einem Verfahren zur Behandlung des menschlichen oder tierischen Körpers bei einer Therapie.

5. Bioaktiver Stoff gemäß Anspruch 4 zur Verwendung bei der Behandlung einer Krankheit, wobei IL-6 beteiligt ist.

6. Bioaktiver Stoff gemäß Anspruch 4 zur Verwendung bei der Behandlung von Krebs Kachexie, multiplem Myelom oder rheumatoider Arthritis.

7. Pharmazeutische Zusammensetzung, die einen bioaktiven Stoff, wie in Anspruch 1 definiert, und ein(en) pharmazeutisch annehmbaren(s) Träger oder Verdünnungsmittel umfaßt.

## Revendications

1. Substance bioactive appelée K93-0711 I-1 ou K93-0711 I-2, qui peut être obtenue en cultivant des *Streptomyces sp.* K93-0711 (FERM BP-5764) et qui possède les propriétés physico-chimiques suivantes :
(1) Substance bioactive K93-0711 I-1 ;
(i) Formule moléculaire : C₂₂H₂₇O₄N (spectre de masse FAB à haute résolution)
(ii) Masse moléculaire : 369 (spectre de masse FAB)
(iii) Spectre d'absorption de la lumière ultraviolette et visible : le spectre UV dans le méthanol présente des maximums d'absorption spécifiques à 299 (log ε 3,28), 245 (log ε 4,26) et 207 (log ε 4,38) nm
(iv) Spectre d'absorption des infrarouges :
Le spectre IR dans KBr présente des bandes d'absorption à 3390, 2927, 1741, 1695, 1603, 1487, 1381, 1281 et 756 cm⁻¹
(v) Rotation spécifique [α]²⁴_{D} +44,4°(c. 0,3, MeOH)
(vi) Solubilité :
Soluble dans l'acétone, l'acétate d'éthyle, l'éther éthylique, l'hexane, le méthanol, l'éthanol, le chloroforme et le benzène
Légèrement soluble dans l'eau
(vii) Spectre ¹H-RMN (dans du chloroforme deutérié, 400 MHz) et spectre ¹³C-RMN (dans du chloroforme deutérié, 100.58 MHz)
| Décalage ¹³C | Décalage ¹H (J) |
|---|---|
| 206,4 (s) | |
| 206,3 (s) | |
| 157,7 (s) | |
| 156,6 (s) | |
| 150,5 (s) | |
| 130,5 (d) | 7,19 (1H, m) |
| 129,5 (s) | |
| 123,5 (d) | 7,19 (1H, m) |
| 119,1 (d) | 6,75 (1H, td, 7,3, 0,5) |
| 108,0 (d) | 6,63 (1H, d, 8,0) |
| 106,2 (d) | 4,93 (1H, s) |
| 88,0 (s) | |
| 66,6 (t) | 3,84 (1H, m) 3,14 (1H, m) |
| 53,7 (t) | 3,69 (1H, d, 14,2) |
| | 3,45 (1H, d, 14,2) |
| 50,6 (s) | |
| 41,1 (t) | 2,33 (1H, dd, 12,0, 9,0) |
| | 2,17 (1H, ddd, 12,0, 5,0, 1,5) |
| 29,9 (t) | 1,29 (2H, m) 1,29 |
| 23,5 (t) | 2,38 (1H, m) 2,36 (1H, m) |
| 22,7 (t) | 1,19 (2H, m) |
| 17,3 (q) | 1,12 (3H, s) |
| 13,8 (q) | 0,76 (3H, t, 7,0) |
| 9,4 (q) | 2,00 (3H, s) |
(viii) Réaction colorée : positive pour l'acide sulfurique, Ehrlich et l'iode.
(2) Substance bioactive K93-0711 I-2 ;
(i) Formule moléculaire : C₂₂H₂₇O₄N (spectre de masse FAB à haute résolution)
(ii) Masse moléculaire : 369 (spectre de masse FAB)
(iii) Spectre d'absorption de la lumière ultraviolette et visible : le spectre UV dans le méthanol présente des maximums d'absorption spécifique à 304 (log ε 3,28), 246 (log ε 4,29) et 208 (log ε 4,40) nm
(iv) Spectre d'absorption des infrarouges :
Le spectre IR dans KBr présente des bandes d'absorption à 3410, 2927, 1738, 1693, 1597, 1489, 1380, 1282 et 756 cm⁻¹
(v) Rotation spécifique [α]²⁴_{D} +25,6° (c. 0,3, MeOH)
(vi) Solubilité :
Soluble dans l'acétone, l'acétate d'éthyle, l'éther éthylique, l'hexane, le méthanol, l'éthanol, le chloroforme et le benzène
Légèrement soluble dans l'eau
(vii) Spectre ¹H-RMN (dans du chloroforme deutérié, 400 MHz) et spectre ¹³C-RMN (dans du chloroforme deutérié, 100.58 MHz)
| Décalage ¹³C | Décalage ¹H (J) |
|---|---|
| 206,6 (s) | |
| 205,9 (s) | |
| 160,3 (s) | |
| 153,7 (s) | |
| 150,4 (s) | |
| 130,5 (d) | 7,19 (1H, m) |
| 129,4 (s) | |
| 123,5 (d) | 7,19 (1H, m) |
| 118,9 (d) | 6,74 (1H, td, 7,2, 1,0) |
| 107,8 (d) | 6,62 (1H, d, 8,0) |
| 105,0 (d) | 4,92 (1H, s) |
| 87,9 (s) | |
| 66,8 (t) | 3,85 (1H, ddd, 12,0, 7,9, 1,5) |
| | 3,17 (1H, ddd, 12,0, 9,2, 5,0) |
| 52,1 (t) | 3,69 (1H, d, 14,9) |
| | 3,49 (1H, d, 14,9) |
| 50,5 (s) | |
| 41,2 (t) | 2,33 (1H, dd, 12,0, 7,9, 1,5) |
| | 2,19 (1H, ddd, 12,0, 9,2, 1,5) |
| 29,5 (t) | 1,39 (2H, m) |
| 24,0 (t) | 2,46 (1H, m) 2,37 (1H, m) |
| 17,5 (q) | 1,12 (3H, s) |
| 13,8 (q) | 0,86 (3H, t, 7,1) |
| 9,1 (q) | 1,94 (3H, s) |
(viii) Réaction colorée : positive pour l'acide sulfurique, Ehrlich et l'iode.

2. Procédé de production de la substance bioactive K93-0711 I-1 ou I-2 selon la revendication 1, comprenant les étapes consistant à cultiver *Streptomyces* sp. K93-0711 (FERM BP-5764), ou un mutant de celui-ci qui produit ladite substance bioactive K93-0711 I-1 ou I-2, dans un milieu, pour accumuler ladite substance bioactive K93-0711 I-1 ou I-2 dans le milieu ; et à isoler ladite substance bioactive à partir de celui-ci.

3. *Streptomyces sp.* K93-0711 (FERM BP-5764), ou mutant de celui-ci qui produit ladite substance bioactive K93-0711 I-1 ou I-2 telle que définie dans la revendication 1.

4. Substance bioactive selon la revendication 1 destinée à être utilisée dans un procédé pour traiter un corps humain ou animal par thérapie.

5. Substance bioactive selon la revendication 4 destinée à être utilisée pour traiter une maladie impliquant IL-6.

6. Substance bioactive selon la revendication 4 destinée à être utilisée pour traiter la cachexie néoplasique, les myélomes multiples et la polyarthrite rhumatoïde.

7. Composition pharmaceutique comprenant une substance bioactive selon la revendication 1 et un véhicule ou diluant pharmaceutiquement acceptable.
